# FASCICULE DE BREVET EUROPEEN

(11) **EP 1 237 599 B1**
(45) Date de publication et mention de la délivrance du brevet: **13.07.2005**
(21) Numéro de dépôt: 00988870.2
(22) Date de dépôt: 23.11.2000
(51) Int. Cl.: A61M 5/30

(54) **SERINGUE SANS AIGUILLE FONCTIONNANT AVEC ENTRAîNEMENT DU PRINCIPE ACTIF PAR EFFET TUBE A CHOC**
NADELLOSE SPRITZE MIT IN STRÖMUNG BEI STOSSWELLENROHREFFEKT GETRAGENEN WIRKSTOFFPARTIKELN
NEEDLELESS SYRINGE WHICH FUNCTIONS WITH THE ACTIVE AGENT BEING DRIVEN BY A SHOCK-TUBE EFFECT

(30) Priorité: 08.12.1999 FR 9915473
(43) Date de publication de la demande: 11.09.2002
(73) Titulaire: Crossject, 75181 Paris Cédex 04 (FR)
(72) Inventeur: ALEXANDRE, Patrick, F-70100 Gray (FR); DELANNOY, Guy, F-33160 Saint Medard en Jalles (FR); GAUTIER, Philippe, F-91220 Le Plessis Pate (FR); ROLLER, Denis, F-91590 La Ferte Alais (FR)
(86) Numéro de dépôt international: PCT/FR2000/003256
(87) Numéro de publication internationale: WO 2001/041839

(56) Documents cités:
- EP-A- 0 755 689
- EP-A- 0 821 195
- FR-A- 2 796 290
- US-A- 4 089 334
- US-A- 5 899 880

## Description

Le domaine technique de l'invention est celui des seringues sans aiguille utilisées pour les injections hypodermiques ou intramusculaires de divers principes actifs pulvérulents ou sous forme de poudres sèches, à usage thérapeutique pour la médecine humaine ou vétérinaire.

Plus spécifiquement, l'invention se rapporte à une seringue sans aiguille mettant en oeuvre un générateur de gaz destiné à créer une onde de pression pour éjecter les particules de principe actif. Un opercule claquable, placé sur le chemin des gaz, permet d'obtenir le niveau de pression seuil permettant d'éjecter les particules avec une vitesse suffisamment élevée. En fait, la libération soudaine des gaz crée dans la seringue un choc thermodynamique et c'est l'onde de choc qui va porter et accélérer les particules afin de les expulser de la seringue. La spécificité de l'invention réside dans le fait que la seringue sans aiguille fonctionne avec une charge pyrotechnique comme source énergétique thermodynamique d'expulsion des particules solides, cette charge pyrotechnique étant étroitement associée à la géométrie du tube d'éjection et à sa dimension.

Les seringues sans aiguille fonctionnant par libération d'un gaz sous pression pour expulser des particules solides de principe actif ont déjà fait l'objet de plusieurs brevets. On peut citer en particulier la demande de brevet WO 94/24263, qui décrit une seringue sans aiguille fonctionnant par libération d'une réserve de gaz, pour entraîner les particules solides de principe actif initialement placées entre deux membranes transversales soufflables, positionnées dans le tube d'éjection des particules mais un tel dispositif encombrant présente des difficultés de mise au point et ne permet que difficilement de créer une onde de choc efficace puisqu'aucun effet thermique ne peut être combiné à la pression des gaz.

En revanche, des seringues sans aiguille impliquant une charge pyrotechnique pour expulser un principe actif ont déjà été mises au point, mais uniquement pour des principes actifs liquides et non pour des principes actifs pulvérulents, aucun effet de tube à choc ne se produisant puisque les gaz ne font que déplacer un piston agissant sur le liquide à expulser.

A titre d'exemple, le brevet US 2 322 244 décrit un injecteur hypodermique sans aiguille fonctionnant à partir d'une cartouche à blanc, dont la mise à feu est provoquée par la percussion d'un piston mis en vitesse par la détente d'un ressort. Le liquide à injecter étant placé au contact de la cartouche, est expulsé de l'injecteur sous l'effet de la pression générée par les gaz de combustion.

Le brevet US 4 089 334, quant à lui, se rapporte à un injecteur sans aiguille muni d'une charge pyrotechnique dont la mise à feu est assurée par une charge primaire, elle-même initiée par percussion d'une tige rigide mise en mouvement par la détente d'un ressort. Les gaz émis par la charge pyrotechnique s'expansent dans une chambre aval, mettant en mouvement un piston dont le déplacement provoque l'expulsion du produit liquide à injecter.

Ces deux brevets étant relatifs à des injecteurs sans aiguille destinés à expulser du principe actif sous forme liquide, les gaz émis par la charge pyrotechnique n'ont qu'une fonction de poussée assimilable à celle d'un piston. Jamais ces brevets ne mettent en avant la création d'une onde de choc pour accélérer des particules solides.

Les seringues sans aiguille, de façon générale, doivent être peu encombrantes, performantes, fiables et doivent pouvoir facilement s'adapter au cas à traiter en terme, par exemple, de profondeur de pénétration des particules de principe actif. L'utilisation de charges pyrotechniques très énergétiques dans une seringue sans aiguille, associée à un opercule calibré, permet de répondre à ces exigences. En effet, par rapport à une réserve de gaz sous pression classique, une charge pyrotechnique va pouvoir produire dans la seringue, un niveau de pression très élevé en un temps très court, qui sont les deux conditions requises pour produire une onde de choc, et il a été découvert que du fait, d'une part, de la détente des gaz pyrotechniques à haute température initiale et, d'autre part, du très faible temps de contact entre ces gaz et le principe actif ainsi qu'entre ces gaz et la zone cutanée d'injection, il n'y a aucun inconvénient à utiliser une charge pyrotechnique génératrice de gaz.

L'onde de choc se caractérise essentiellement par sa brièveté, sa très forte intensité et sa très grande vitesse de déplacement qui sont des caractéristiques motrices particulièrement adaptées pour injecter des particules solides à très haute vitesse, de l'ordre de 800 m/s. De plus, les mises à feu de charges pyrotechniques étant parfaitement maîtrisées avec des dispositifs d'initiation largement éprouvés comme ceux pouvant, par exemple, impliquer une charge primaire ou une amorce, les seringues sans aiguille selon l'invention présentent au niveau de leur fonctionnement moteur une très grande fiabilité.

Un autre point à souligner, concernant les avantages liés à l'utilisation d'une charge pyrotechnique, porte sur la grande variabilité des compositions pouvant intégrer le dispositif de déclenchement de la seringue, permettant ainsi d'ajuster les paramètres du moteur de ladite seringue au cas à traiter. Enfin, dans leur phase de stockage, les seringues sans aiguille selon l'invention ne sont pas pressurisées comme peuvent l'être celles fonctionnant avec une réserve de gaz sous pression, permettant, d'une part, de réduire de façon significative, les risques d'incidents et, d'autre part, d'assurer une grande stabilité dans le temps du moteur de la seringue.

Les seringues sans aiguille selon l'invention possèdent toutes ces caractéristiques.

L'objet de la présente invention concerne une seringue sans aiguille comprenant successivement un générateur de gaz, une chambre d'expansion des gaz, un moyen de retenue des particules d'un principe actif et un tube d'éjection desdites particules, caractérisée en ce que le générateur de gaz est un générateur pyrotechnique qui comprend une charge pyrotechnique génératrice de gaz et un dispositif d'initiation, et en ce que le moyen de retenue comprend au moins un opercule fixé au tube d'éjection et destiné à se rompre sous l'effet des gaz produits par la combustion de ladite charge. Le phénomène recherché par l'utilisation d'une charge pyrotechnique est la création d'une onde de choc pour emporter les particules solides de principe actif et les expulser avec une vitesse suffisante, vers la peau du patient à traiter. L'onde de choc résulte de la mise en pression très rapide des gaz dans un volume constant représenté par la chambre d'expansion et de la rupture d'un opercule calibré provoquant la libération quasi-instantanée des gaz. La géométrie, l'intensité et la vitesse de l'onde de choc ainsi produite, sont fonction des caractéristiques de la charge pyrotechnique utilisée, de la forme et du volume de la chambre d'expansion ainsi que de la forme et des dimensions du tube d'éjection. Préférentiellement, la charge pyrotechnique est un propergol peu oxygéné pour lequel la combustion vive produit des gaz dits «froids», dont la température est inférieure à 1900 K, comme par exemple, des propergols à base d'azoture de sodium ou de nitrate d'ammonium. De façon plus générale, le propergol a une composition non hydrocarbonée et fortement azotée, ou une composition hydrocarbonée oxygénée.

Structurellement, le propergol peut se présenter soit sous la forme d'un bloc compact soit sous une forme divisée. Il est également possible de choisir une poudre dont la quasi-déflagration contrôlée permettra l'obtention des mêmes caractéristiques.

En effet, les effets engendrés par la combustion d'une poudre ou d'un propergol se résument surtout à la production de gaz, ceux-ci se caractérisant essentiellement par leur composition, leur température et la pression à laquelle ils sont émis. Il est donc souhaitable de choisir des compositions pyrotechniques dont la combustion ne va directement ni altérer les particules de principe actif retenues dans la seringue, ni présenter une quelconque source de danger pour le patient à traiter, mais il est toujours possible d'interposer un refroidisseur de type puits thermique entre la charge pyrotechnique génératrice de gaz et le principe actif, un tel puits thermique pouvant, par exemple, être constitué par des sphérules d'alumine disposées dans un filtre constitué par de fines grilles métalliques. C'est pour ces raisons que les charges pyrotechniques retenues doivent produire des gaz peu toxiques, ayant une température relativement peu élevée. Il faut toutefois préciser que la température des gaz ne doit pas être considérée comme le paramètre déterminant au niveau du danger présenté par la seringue vis à vis du patient à traiter, puisque les gaz sont émis pendant un temps très bref, de l'ordre de quelques millisecondes, rendant caduque l'agression par les flux thermiques de la peau du patient.

De façon avantageuse, l'opercule est calibré pour céder à une pression dynamique dans la chambre d'au moins 70 bars et, préférentiellement, à une pression dynamique comprise entre 80 bars et 200 bars. De façon préférentielle, la chambre d'expansion comporte un filtre ayant la double fonction de retenir les particules indésirables issues des gaz de combustion, et de refroidir également lesdits gaz. Avantageusement, la température maximale atteinte dans la chambre d'expansion est comprise entre 350 K et 1500 K. Préférentiellement, le tube d'éjection est un cylindre droit.

Selon un premier mode de réalisation préféré de l'invention, la chambre d'expansion des gaz est sensiblement cylindrique et son diamètre interne est voisin de celui du tube d'éjection. De façon avantageuse, le rapport de la somme des longueurs de la chambre et du tube sur leur diamètre est compris entre 3 et 25, et préférentiellement entre 7 et 18.

Selon un second mode de réalisation préféré de l'invention, la chambre d'expansion présente une forme sensiblement cylindrique prolongée par une zone de rétrécissement débouchant sur le tube d'éjection, de sorte que le diamètre interne dudit tube est inférieur au diamètre interne de la partie cylindrique de ladite chambre et l'opercule est fixé dans le tube d'éjection de diamètre réduit.

De façon préférentielle, la zone de rétrécissement est progressive en ayant une forme de convergent de tuyère. En effet, le passage d'une configuration où les diamètres de la chambre et du tube sont identiques à une configuration pour laquelle le diamètre du tube est inférieur à celui de la chambre, s'accompagne toujours d'un accroissement des vitesses d'éjection des particules de principe actif, et ce, pour une même charge pyrotechnique. De façon avantageuse, le rapport du diamètre de la partie cylindrique de la chambre d'expansion sur le diamètre interne du tube d'éjection est compris entre 1,1 et 3, et préférentiellement entre 1,3 et 2,5.

Avantageusement, le segment aval du tube, par lequel sont éjectées les particules, présente une partie conique divergente prolongée par une partie cylindrique droite dont l'extrémité libre vient au contact de la peau. De cette manière, cette partie divergente permet d'augmenter la surface de l'évent et donc de diminuer la pression en sortie de seringue sans réduire la vitesse d'éjection des particules.

Selon l'un ou l'autre des deux modes de réalisation préférés de l'invention précédents, le rapport de la longueur du tube sur la longueur de la chambre est compris entre 1 et 5 et la somme de ces deux longueurs est comprise entre 8 cm et 15 cm. Préférentiellement, la longueur de la chambre vaut 3,5 cm et celle du tube vaut 8,5 cm.

Avantageusement, le diamètre des particules de principe actif est compris entre 20µm et 100µm et préférentiellement entre 50µm et 80µm, et la masse totale dudit principe actif est comprise entre 1 mg et 10 mg et préférentiellement entre 2 mg et 7 mg. De façon avantageuse, les particules sont logées entre l'opercule et une membrane placée en aval dudit opercule par rapport au sens de propagation des gaz. Préférentiellement, ladite membrane est fine, inélastique et transversale par rapport à l'axe du tube. Avantageusement, le compactage des particules est compris entre 1% et 70%, et préférentiellement entre 10% et 50%. Le compactage se définit comme étant le rapport du volume total des particules sur le volume total du tube d'éjection compris entre l'opercule et la membrane.

De façon préférentielle, la densité des particules de principe actif est comprise entre 1 et 22, et préférentiellement entre 3 et 18. En fait, c'est la combinaison des deux paramètres « diamètre des particules » et « densité des particules » qui va conditionner leur vitesse d'éjection. En théorie, la vitesse des particules est inversement proportionnelle à la densité et au carré du diamètre. Par calcul, il a été montré que des particules de petit diamètre peuvent avoir des densités élevées sans pour autant affecter, de façon significative, leur vitesse. En revanche, si les particules ont une taille importante tout en ayant une densité également importante, le risque à redouter est que l'onde de choc, issue de l'opercule qui se déchire, traverse ces particules à forte inertie, sans véritablement les porter tout le long de leur trajet, avec pour conséquence majeure une décélération des particules par rapport aux gaz d'entraînement, et en définitive, une vitesse d'impact sur la peau trop faible pour permettre leur pénétration.

De façon avantageuse, le rapport de la masse de la charge pyrotechnique sur la masse de principe actif à éjecter est compris entre 1 et 50, et préférentiellement entre 5 et 40. Selon un autre mode de réalisation préféré de l'invention, le moyen de retenue des particules comporte une grille transversale fixée au tube d'éjection et sur laquelle sont maintenues lesdites particules, celles-ci étant susceptibles d'être éjectées sous l'effet de la pression des gaz engendrés par la combustion de la charge pyrotechnique. Cette grille reste fixée à l'intérieur de la seringue après le passage de l'onde de choc et permet ainsi d'éviter tout risque de projections parasites par rupture de matière.

Avantageusement, le dispositif d'initiation de la charge pyrotechnique comprend un dispositif de percussion et une amorce couramment utilisés en industrie pyrotechnique. Mais il est également possible d'initier la charge pyrotechnique par d'autres moyens, et notamment, ceux impliquant soit un cristal piézoélectrique, soit un rugueux ou même une pile. Il est rappelé qu'un rugueux est une petite pièce rugueuse sur laquelle peut venir frotter un élément sensible à la friction, dans un dispositif pyrotechnique.

Préférentiellement, la seringue dispose d'un déclencheur sous la forme d'un bouton-poussoir placé à l'une de ses extrémités pour faciliter sa préhension et son fonctionnement.

Les seringues sans aiguille selon l'invention bénéficient des avantages liés à un fonctionnement par onde de choc, en particulier en terme de vitesse d'éjection des particules, tout en assurant un maintien fiable des particules en mode stockage.

De plus, par rapport à une source de gaz sous pression classique, une charge pyrotechnique produit des effets au moins aussi intenses, surtout en terme de pression, tout en étant d'encombrement beaucoup plus réduit.

La mise à feu des charges pyrotechniques quelles que soient leur nature et leur dimension est, d'autre part, parfaitement maîtrisée par l'utilisation d'initiateurs largement éprouvés, rendant ainsi la partie motrice de la seringue très fiable et peu coûteuse.

Enfin, les seringues sans aiguille selon l'invention sont optimisées en performance à partir d'une géométrie judicieuse du tube d'éjection.

On donne, ci-après, la description détaillée de trois modes de réalisation préférés de l'invention en se référant aux figures 1 à 3.
La figure 1 est une vue en coupe axiale longitudinale d'une seringue sans aiguille selon l'invention pour laquelle les diamètres de la chambre d'expansion et du tube d'éjection sont identiques.
La figure 2 est un schéma en coupe axiale longitudinale d'une chambre d'expansion présentant une zone de rétrécissement prolongée par un tube d'éjection cylindrique droit.
La figure 3 est un schéma en coupe axiale longitudinale d'une chambre d'expansion analogue à celle de la figure 2 pour laquelle le tube d'éjection présente une partie terminale divergente.

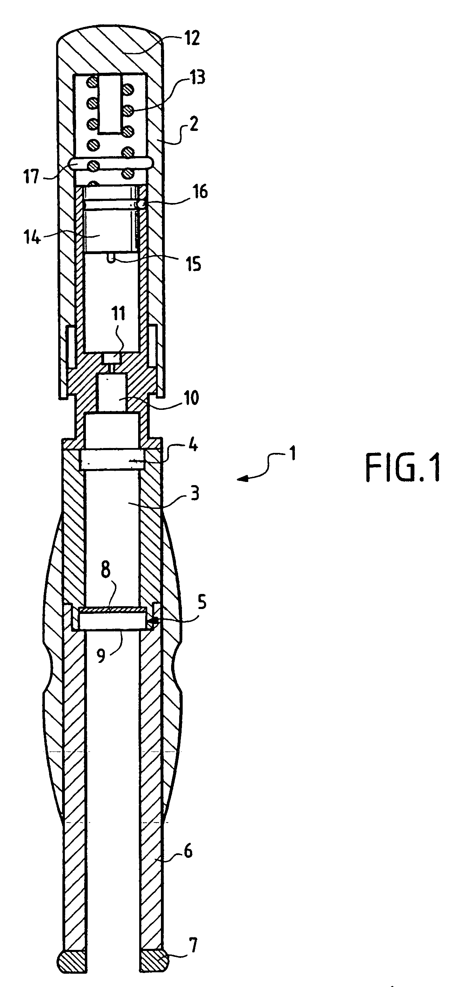

En se référant à la figure 1, une seringue sans aiguille 1 selon l'invention comprend successivement un générateur de gaz pyrotechnique 2, une chambre d'expansion 3 munie d'un filtre 4, un système de retenue 5 des particules et le tube d'éjection 6 desdites particules destiné à venir en appui contre la peau du patient à traiter.

Préférentiellement, cette mise en appui peut être facilitée par un bourrelet 7 amortissant situé à l'extrémité dudit tube 6. La chambre d'expansion 3 des gaz ainsi que le canal interne du tube d'éjection 6 sont sensiblement de forme cylindrique et ont tous les deux le même diamètre. Le système de retenue 5 des particules qui marque la frontière entre la chambre 3 et le tube 6 est constitué par un opercule claquable 8 et une membrane légère 9 placée en aval dudit opercule, ces deux éléments étant parallèles entre eux, en position transversale par rapport à l'axe du tube 6 et fixés tous les deux audit tube 6. Les particules de principe actif occupent l'espace délimité par ces deux éléments, avec un taux de compactage préférentiellement compris entre 1% et 70%.

Selon un mode de réalisation préféré de l'invention, la longueur de la chambre 3 est de 3,5 cm, la longueur du tube d'éjection 6 est de 8,5 cm et leur diamètre vaut 0,8 cm. L'opercule claquable, qui est situé du côté de la chambre d'expansion 3, est calibré pour se rompre à une pression dynamique au moins égale à 70 bars et la membrane 9, quant à elle, sert uniquement de maintien aux particules, sans présenter le moindre caractère de résistance vis à vis des gaz produits.

Avantageusement, ladite membrane 9 est fine et inélastique et présente, comme l'opercule 8, des lignes d'affaiblissement définissant un motif étoilé pour permettre une ouverture en pétales, sans risquer de provoquer un morcellement désordonné pouvant conduire à la production de morceaux parasites.

Selon un autre mode de réalisation, la membrane 9 peut être remplacée par une grille transversale également fixée à l'intérieur du tube 6 et contenant, insérées dans ses interstices, les particules de principe actif. Par rapport au sens de propagation des gaz émis, l'opercule 8 reste en amont de ladite grille.

La chambre d'expansion 3 possède à son extrémité la plus proche du générateur pyrotechnique 2 de gaz un filtre 4 transversal destiné, d'une part, à piéger certaines particules solides issues de la combustion et, d'autre part, à refroidir les gaz avant qu'ils n'entrent dans ladite chambre 3. Avantageusement, ledit filtre 4 est constitué d'un empilement de grilles métalliques avec un pas de plus en plus rapproché et se terminant par une feuille de papier céramique.

Ce filtre 4 permet à la température des gaz de ne pas excéder 1500 k dans la chambre d'expansion 3, de façon à ne pas altérer les particules de principe actif disposées dans leur logement. Le générateur pyrotechnique 2 de gaz comprend un dispositif d'initiation de la charge pyrotechnique 10 faisant intervenir un dispositif de percussion et une amorce 11. Le dispositif de percussion, qui est déclenché par un bouton poussoir 12, comprend un ressort 13 et une masselotte 14 munie d'un percuteur 15. La masselotte 14 est bloquée par au moins une bille 16 coincée entre ladite masselotte 14 et le bouton poussoir 12 et ledit bouton poussoir 12 possède une gorge interne 17 circulaire.

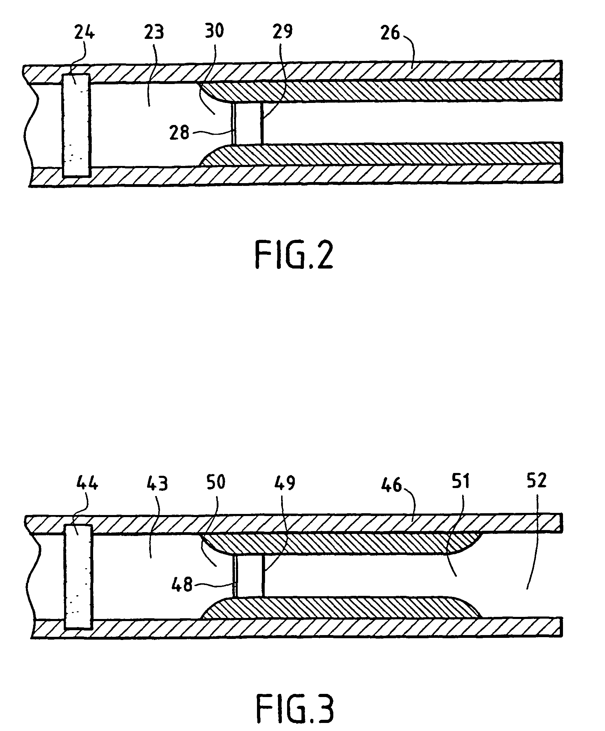

En se référant à la figure 2, selon un deuxième mode de réalisation préféré de l'invention, la seringue sans aiguille comprend successivement un générateur pyrotechnique de gaz non représenté sur la figure, une chambre d'expansion 23 des gaz, un moyen de retenue des particules constitué également par un opercule claquable 28 et une membrane 29 placée en aval dudit opercule 28, et un tube d'éjection 26 desdites particules. La chambre 23 possède un filtre 24 ayant les mêmes fonctions que celles décrites pour le premier mode de réalisation préféré de l'invention, à savoir, capture des particules solides indésirables et refroidissement des gaz de combustion.

Selon ce mode de réalisation préféré de l'invention, la seringue possède le même générateur de gaz pyrotechnique que celui décrit succinctement pour le premier mode de réalisation préféré de l'invention. La principale différence avec le premier mode de réalisation ci avant décrit, réside dans le fait que le tube d'éjection 26 a un diamètre interne réduit, inférieur à celui de la chambre d'expansion 23. Plus précisément, la chambre d'expansion 23 présente une forme sensiblement cylindrique prolongée par une zone de rétrécissement 30 progressive débouchant sur le tube d'éjection 26.

Le système de retenue des particules est situé dans ledit tube 26, juste à la sortie de la zone de rétrécissement 30 de la chambre 23 correspondant à l'endroit où le tube 26 commence à avoir une section constante.

Préférentiellement, la somme des longueur de la chambre 23 et du tube 26 vaut 10 cm et les diamètres de la chambre 23 et du tube 26 valent respectivement 1,2 cm et 0,8 cm. Préférentiellement, la zone de rétrécissement 30 a la forme d'un convergent de tuyère et sa longueur vaut 0,6 cm. Pour un générateur de gaz pyrotechnique donné, la configuration pour laquelle la section du tube 26 est inférieure à celle de la chambre 23 est plus performante, en terme de vitesse d'émission de particules de principe actif, que celle pour laquelle la chambre 3 et le tube 6 sont en continuité l'un de l'autre avec le même diamètre.

En se référant à la figure 3, selon un troisième mode de réalisation préféré de l'invention, la seringue sans aiguille comprend successivement un générateur pyrotechnique de gaz non représenté sur la figure, une chambre d'expansion 43 des gaz, un moyen de retenue des particules constitué également par un opercule claquable 48 et une membrane 49, et un tube d'éjection 46 desdites particules. La chambre 43 possède un filtre 44 ayant les mêmes fonctions que celles décrites précédemment. Le tube d'éjection 46 a un diamètre réduit inférieur à celui de la chambre d'expansion 43, ladite chambre 43 présentant une forme sensiblement cylindrique prolongée par une zone de rétrécissement 50 progressive débouchant sur le tube d'éjection 46. Le système de retenue des particules est situé au même endroit que celui précisé dans la description du deuxième mode de réalisation préféré de l'invention. La différence fondamentale avec le deuxième mode de réalisation préféré de l'invention est que le segment aval du tube 46 par lequel sont éjectées les particules présente une partie conique divergente 51 prolongée par une partie cylindrique droite 52 dont l'extrémité libre vient au contact de la peau du patient à traiter. Cet évasement terminal constitue un évent pour la surpression produite dans le tube 46 et a pour fonction principale de disperser la pression résiduelle en sortie de seringue, de façon à diminuer toute sollicitation indésirable pouvant être néfaste pour le patient. Cette chute de pression n'affecte pratiquement pas la vitesse des particules au moment où elles vont impacter la peau.

Les caractéristiques dimensionnelles du deuxième mode de réalisation de l'invention sont conservées pour ce troisième mode de réalisation, en précisant en plus que la longueur de la zone conique divergente 51 du tube 46 vaut approximativement 0,8 cm.

Le principe de fonctionnement d'une seringue sans aiguille selon l'invention suit les étapes suivantes.

L'utilisateur positionne la seringue 1 de façon à ce que l'extrémité du tube d'éjection 6, 26, 46 vienne en appui contre la peau du patient à traiter. Une pression sur le bouton poussoir 12 permet de le faire coulisser le long de la seringue jusqu'à ce que la gorge 17 vienne au niveau de la bille 16 qui bloque la masselotte 14. Un ressort 13 placé dans la seringue 1 confère une certaine résistance au bouton poussoir 12 de façon à obliger l'utilisateur à produire un effort particulier pour enfoncer ledit bouton 12. La bille 16 n'étant alors plus coincée, libère la masselotte 14 qui, sous l'effet du ressort 13 qui se détend, est propulsée vers l'amorce 11, le percuteur 15 en avant.

L'amorce 11 qui est alors initiée provoque la mise à feu de la charge pyrotechnique 10. Les gaz de combustion passent d'abord à travers un filtre 4, 24, 44 qui retient une partie de leurs particules et les refroidit, puis, s'accumulent dans la chambre d'expansion 3, 23, 43 jusqu'à atteindre une pression seuil située aux alentours de 70 bars.

L'opercule 8, 28, 48 se rompt brutalement, créant une onde de choc dont le front met en vitesse les particules de principe actif juste retenues par une membrane 9, 29, 49 non résistante. Les particules solides de principe actif sont alors accélérées dans le tube d'éjection 6, 26, 26 avant d'impacter la peau du patient à traiter.

## Revendications

1. Seringue sans aiguille (1) comprenant successivement un générateur de gaz, une chambre (3, 23, 43) d'expansion des gaz, un moyen de retenue des particules d'un principe actif et un tube d'éjection (6, 26, 46) desdites particules, **caractérisée en ce que** le générateur de gaz est un générateur pyrotechnique (2) qui comprend une charge pyrotechnique (10) génératrice de gaz et un dispositif d'initiation, et **en ce que** le moyen de retenue comprend au moins un opercule (8, 28, 48) fixé au tube d'éjection (6, 26, 46) et destiné à se rompre sous l'effet des gaz produits par la combustion de ladite charge (10).

2. Seringue sans aiguille selon la revendication 1, **caractérisée en ce que** la charge pyrotechnique (10) est un propergol peu oxygéné produisant des gaz dont la température est inférieure à 1900 K.

3. Seringue sans aiguille selon la revendication 1, **caractérisée en ce que** l'opercule (8, 28, 48) est calibré pour céder à une pression dynamique dans la chambre (3, 23, 43) d'au moins 70 bars.

4. Seringue sans aiguille selon la revendication 1, **caractérisée en ce que** la chambre d'expansion (3, 23, 43) comporte un filtre (4, 24, 44).

5. Seringue sans aiguille selon l'une quelconque des revendications 1 ou 2, **caractérisée en ce que** la température maximale atteinte dans la chambre d'expansion (3, 23, 43) est comprise entre 350 K et 1500 K.

6. Seringue sans aiguille selon l'une quelconque des revendications 1 ou 2, **caractérisée en ce que** le tube d'éjection (6) est un cylindre droit.

7. Seringue sans aiguille selon la revendication 6, **caractérisée en ce que** la chambre d'expansion (3) des gaz est sensiblement cylindrique et son diamètre interne est voisin de celui du tube d'éjection (6).

8. Seringue sans aiguille selon la revendication 7, **caractérisée en ce que** le rapport de la somme des longueurs de la chambre (3) et du tube (6) sur leur diamètre est compris entre 3 et 25.

9. Seringue sans aiguille selon la revendication 6, **caractérisée en ce que** la chambre d'expansion (23) présente une forme sensiblement cylindrique prolongée par une zone de rétrécissement (30) débouchant sur le tube d'éjection (26), de sorte que le diamètre interne dudit tube (26) est inférieur au diamètre interne de la partie cylindrique de ladite chambre (23) et l'opercule (28) est fixé dans le tube d'éjection (26) de diamètre réduit.

10. Seringue sans aiguille selon la revendication 9, **caractérisée en ce que** la zone de rétrécissement (30) est progressive en ayant une forme de convergent de tuyère.

11. Seringue sans aiguille selon la revendication 9, **caractérisée en ce que** le rapport du diamètre de la partie cylindrique de la chambre d'expansion (23) sur le diamètre interne du tube d'éjection (26) est compris entre 1,1 et 3.

12. Seringue sans aiguille selon la revendication 9, **caractérisée en ce que** le segment aval du tube (46), par lequel sont éjectées les particules, présente une partie conique divergente (51) prolongée par une partie cylindrique droite (52) dont l'extrémité libre vient au contact de la peau.

13. Seringue sans aiguille selon l'une quelconque des revendications 7, 9 ou 12, **caractérisée en ce que** le rapport de la longueur du tube (6, 26, 46) sur la longueur de la chambre (3, 23, 43) est compris entre 1 et 5 et la somme de ces deux longueurs est comprise entre 8 cm et 15 cm.

14. Seringue sans aiguille selon l'une quelconque des revendications 7 ou 9, **caractérisée en ce que** le diamètre des particules de principe actif est compris entre 20µm et 100µm et la masse totale dudit principe actif est comprise entre 1 mg et 10 mg.

15. Seringue sans aiguille selon la revendication 1, **caractérisée en ce que** les particules sont logées entre l'opercule (8, 28, 48) et une membrane (9, 29, 49) placée en aval dudit opercule (8, 28, 48) par rapport au sens de propagation des gaz.

16. Seringue sans aiguille selon la revendication 15, **caractérisée en ce que** le compactage des particules est compris entre 1% et 70%.

17. Seringue sans aiguille selon la revendication 1, **caractérisée en ce que** la densité des particules est comprise entre 1 et 22.

18. Seringue sans aiguille selon la revendication 1, **caractérisée en ce que** le rapport de la masse de la charge pyrotechnique (10) sur la masse du principe actif à éjecter est compris entre 1 et 50.

19. Seringue sans aiguille selon la revendication 1, **caractérisée en ce que** le moyen de retenue des particules comporte une grille transversale fixée au tube d'éjection et sur laquelle sont maintenues lesdites particules, celles-ci étant susceptibles d'être éjectées sous l'effet de la pression des gaz engendrés par la combustion de la charge pyrotechnique.

20. Seringue sans aiguille selon la revendication 1, **caractérisée en ce que** le dispositif d'initiation de la charge pyrotechnique (10) comprend un dispositif de percussion et une amorce (11).

## Patentansprüche

1. Nadellose Spritze (1), die nacheinander einen Gasgenerator, eine Ausdehnungskammer (3, 23, 43) für die Gase, ein Rückhaltemittel für die Teilchen eines Wirkstoffs und ein Rohr (6, 26, 46) zum Ausstoßen dieser Teilchen aufweist, **dadurch gekennzeichnet, dass** der Gasgenerator ein pyrotechnischer Gasgenerator (2) ist, der eine gaserzeugende pyrotechnische Ladung (10) und eine Zündvorrichtung enthält, und dass das Rückhaltemittel mindestens eine Abdeckfolie (8, 28, 48) aufweist, die am Ausstoßrohr (6, 26, 46) befestigt und dazu bestimmt ist, unter der Wirkung der von der Verbrennung der Ladung (10) erzeugten Gase zu reißen.

2. Nadellose Spritze nach Anspruch 1, **dadurch gekennzeichnet, dass** die pyrotechnische Ladung (10) ein sauerstoffarmes Propergol ist, das Gase erzeugt, deren Temperatur unter 1900 K liegt.

3. Nadellose Spritze nach Anspruch 1, **dadurch gekennzeichnet, dass** die Abdeckfolie (8, 28, 48) so kalibriert ist, dass sie bei einem dynamischen Druck in der Kammer (3, 23, 43) von mindestens 70 Bar nachgibt.

4. Nadellose Spritze nach Anspruch 1, **dadurch gekennzeichnet, dass** die Ausdehnungskammer (3, 23, 43) ein Filter (4, 24, 44) aufweist.

5. Nadellose Spritze nach einem der Ansprüche 1 oder 2, **dadurch gekennzeichnet, dass** die maximal in der Ausdehnungskammer (3, 23, 43) erreichte Temperatur zwischen 350 K und 1500 K liegt.

6. Nadellose Spritze nach einem der Ansprüche 1 oder 2, **dadurch gekennzeichnet, dass** das Ausstoßrohr (6) ein gerader Zylinder ist.

7. Nadellose Spritze nach Anspruch 6, **dadurch gekennzeichnet, dass** die Ausdehnungskammer (3) der Gase im Wesentlichen zylindrisch und ihr Innendurchmesser nahe demjenigen des Ausstoßrohrs (6) ist.

8. Nadellose Spritze nach Anspruch 7, **dadurch gekennzeichnet, dass** das Verhältnis der Summe der Längen der Kammer (3) und des Rohrs (6) zu ihrem Durchmesser zwischen 3 und 25 liegt.

9. Nadellose Spritze nach Anspruch 6, **dadurch gekennzeichnet, dass** die Ausdehnungskammer (23) eine im Wesentlichen zylindrische Form aufweist, die durch eine Verengungszone (30) verlängert wird, die am Ausstoßrohr (26) mündet, so dass der Innendurchmesser des Rohrs (26) kleiner ist als der Innendurchmesser des zylindrischen Bereichs der Kammer (23), und dass die Abdeckfolie (28) im Ausstoßrohr (26) mit verringertem Durchmesser befestigt ist.

10. Nadellose Spritze nach Anspruch 9, **dadurch gekennzeichnet, dass** die Verengungszone (30) progressiv ist und eine konvergierende Düsenform hat.

11. Nadellose Spritze nach Anspruch 9, **dadurch gekennzeichnet, dass** das Verhältnis zwischen dem Durchmesser des zylindrischen Bereichs der Ausdehnungskammer (23) und dem Innendurchmesser des Ausstoßrohrs (26) zwischen 1,1 und 3 liegt.

12. Nadellose Spritze nach Anspruch 9, **dadurch gekennzeichnet, dass** das hintere Segment des Rohrs (46), durch das die Teilchen ausgestoßen werden, einen divergierenden kegelförmigen Bereich (51) aufweist, der von einem geraden zylindrischen Bereich (52) verlängert wird, dessen freies Ende mit der Haut in Kontakt kommt.

13. Nadellose Spritze nach einem der Ansprüche 7, 9 oder 12, **dadurch gekennzeichnet, dass** das Verhältnis zwischen der Länge des Rohrs (6, 26, 46) und der Länge der Kammer (3, 23, 43) zwischen 1 und 5 und die Summe dieser beiden Längen zwischen 8 cm und 15 cm liegt.

14. Nadellose Spritze nach einem der Ansprüche 7 oder 9, **dadurch gekennzeichnet, dass** der Durchmesser der Wirkstoffteilchen zwischen 20 µm und 100 µm und die Gesamtmasse des Wirkstoffs zwischen 1 mg und 10 mg liegt.

15. Nadellose Spritze nach Anspruch 1, **dadurch gekennzeichnet, dass** die Teilchen zwischen der Abdeckfolie (8, 28; 48) und einer Membran (9, 29, 49) angeordnet sind, die sich bezüglich der Ausbreitungsrichtung der Gase hinter der Abdeckfolie (8, 28, 48) befindet.

16. Nadellose Spritze nach Anspruch 15, **dadurch gekennzeichnet, dass** die Verdichtung der Teilchen zwischen 1 % und 70 % liegt.

17. Nadellose Spritze nach Anspruch 1, **dadurch gekennzeichnet, dass** die Dichte der Teilchen zwischen 1 und 22 liegt.

18. Nadellose Spritze nach Anspruch 1, **dadurch gekennzeichnet, dass** das Masseverhältnis zwischen der pyrotechnischen Ladung (10) und der Masse des auszustoßenden Wirkstoffs zwischen 1 und 50 liegt.

19. Nadellose Spritze nach Anspruch 1, **dadurch gekennzeichnet, dass** das Rückhaltemittel für die Teilchen ein Quergitter aufweist, das am Ausstoßrohr befestigt ist und auf dem die Teilchen gehalten werden, die unter der Wirkung des Drucks der Gase ausgestoßen werden können, die von der Verbrennung der pyrotechnischen Ladung erzeugt werden.

20. Nadellose Spritze nach Anspruch 1, **dadurch gekennzeichnet, dass** die Zündvorrichtung der pyrotechnischen Ladung (10) eine Schlagvorrichtung und einen Zünder (11) aufweist.

## Claims

1. Needle-less syringe (1) comprising, in succession: a gas-generator; a chamber (3, 23, 43) for the expansion of the gases; a means for retaining the particles of an active principle; and a tube (6, 26, 46) for ejecting the said particles, **characterised in that** the gas-generator is a pyrotechnic generator (2) which comprises a gas-generating pyrotechnic charge (10) and an initiating device, and **in that** the retaining means comprises at least one cap (8, 28, 48) fixed to the ejection tube (6, 26, 46) and intended to break under the effect of the gases produced by combustion of the said charge (10).

2. Needle-less syringe according to claim 1, **characterised in that** the pyrotechnic charge (10) is a barely oxygenated propellant producing gases whose temperature is lower than 1900 K.

3. Needle-less syringe according to claim 1, **characterised in that** the cap (8, 28, 48) is calibrated so as to give way at a dynamic pressure in the chamber (3, 23, 43) of at least 70 bars.

4. Needle-less syringe according to claim 1, **characterised in that** the expansion chamber (3, 23, 43) has a filter (4, 24, 44).

5. Needle-less syringe according to any of claims 1 or 2, **characterised in that** the maximum temperature reached in the expansion chamber (3, 23, 43) is between 350 K and 1500 K.

6. Needle-less syringe according to any of claims 1 or 2, **characterised in that** the ejection tube (6) is a straight cylinder.

7. Needle-less syringe according to claim 6, **characterised in that** the chamber (3) for the expansion of the gases is substantially cylindrical and its internal diameter is close to that of the ejection tube (6).

8. Needle-less syringe according to claim 7, **characterised in that** the ratio of the sum of the lengths of the chamber (3) and tube (6) over their diameter is between 3 and 25.

9. Needle-less syringe according to claim 6, **characterised in that** the expansion chamber (23) has a substantially cylindrical shape prolonged by a zone of contraction (30) emerging onto the ejection tube (26), in such a way that the internal diameter of the said tube (26) is smaller than the internal diameter of the cylindrical part of the said chamber (23) and the cap (28) is fixed in the ejection tube (26) of reduced diameter.

10. Needle-less syringe according to claim 9, **characterised in that** the zone of contraction (30) is progressive while having the shape of a convergent of a nozzle.

11. Needle-less syringe according to claim 9, **characterised in that** the ratio of the diameter of the cylindrical part of the expansion chamber (23) over the internal diameter of the ejection tube (26) is between 1.1 and 3.

12. Needle-less syringe according to claim 9, **characterised in that** the downstream segment of the tube (46), through which the particles are ejected, has a divergent conical part (51) prolonged by a straight cylindrical part (52) whose free end comes into contact with the skin.

13. Needle-less syringe according to any of claims 7, 9 or 12, **characterised in that** the ratio of the length of the tube (6, 26, 46) over the length of the chamber (3, 23, 43) is between 1 and 5 and the sum of these two lengths is between 8 cm and 15 cm.

14. Needle-less syringe according to any of claims 7 or 9, **characterised in that** the diameter of the particles of active principle is between 20µm and 100µm and the total mass of the said active principle is between 1 mg and 10 mg.

15. Needle-less syringe according to claim 1, **characterised in that** the particles are accommodated between the cap (8, 28, 48) and a diaphragm (9, 29, 49) placed downstream of the said cap (8, 28, 48) in relation to the direction of propagation of the gases.

16. Needle-less syringe according to claim 15, **characterised in that** the compaction of the particles is between 1% and 70%.

17. Needle-less syringe according to claim 1, **characterised in that** the density of the particles is between 1 and 22.

18. Needle-less syringe according to claim 1, **characterised in that** the ratio of the mass of the pyrotechnic charge (10) over the mass of the active principle to be ejected is between 1 and 50.

19. Needle-less syringe according to claim 1, **characterised in that** the means for retaining the particles has a transverse grille which is fixed to the ejection tube and on which the said particles are held, the latter being capable of being ejected under the effect of the pressure of the gases developed by combustion of the pyrotechnic charge.

20. Needle-less syringe according to claim 1, **characterised in that** the device for initiating the pyrotechnic charge 10) comprises a percussion device and a detonator (11).
